# EUROPEAN PATENT SPECIFICATION

(11) **EP 3 601 621 B1**
(45) Date of publication and mention of the grant of the patent: **06.09.2023**
(21) Application number: 18772698.9
(22) Date of filing: 13.03.2018
(51) Int. Cl.: C13K 1/02, C22C 19/00, C22C 38/08, D21C 1/04, D21C 3/04, D21C 7/04

(54) **APPARATUS AND METHOD FOR HYDROLYSIS OF LIGNOCELLULOSIC MATERIALS**
VORRICHTUNG UND VERFAHREN ZUR HYDROLYSE VON LIGNOCELLULOSESTOFFEN
APPAREIL ET PROCÉDÉ POUR L'HYDROLYSE DE MATIÈRES LIGNOCELLULOSIQUES

(30) Priority: 21.03.2017 SE 1750334
(43) Date of publication of application: 05.02.2020
(73) Proprietor: Valmet AB, 851 94 Sundsvall (SE)
(72) Inventor: WALLIN, Göran, 852 36 Sundsvall (SE)
(74) Representative: Novagraaf Group
(86) International application number: PCT/SE2018/050242
(87) International publication number: WO 2018/174778

(56) References cited:
- EP-B1- 2 132 351
- CN-A- 101 603 065
- US-A- 5 494 636
- US-A1- 2007 148 751
- US-A1- 2012 156 748
- US-A1- 2012 279 496
- US-A1- 2013 252 302
- US-A1- 2016 312 249
- A Aden ET AL: "Lignocellulosic biomass to ethanol process design and economics utilizing co-current dilute acid prehydrolysis and enzymatic hydrolysis for corn stover", NREL technical report NREL/TP-510-32438, 1 June 2002 (2002-06-01), page i-iii, 20-36, XP55074470, Retrieved from the Internet: URL:http://www.nrel.gov/docs/fy02osti/3243 8.pdf [retrieved on 2013-08-07]

## Description

### TECHNICAL FIELD

The present invention relates generally to an apparatus, e.g. a reactor vessel, in which extraction of saccharides from a lignocellulosic material is performed by means of hydrolysis of the lignocellulosic material in an acidic water solution, and in particular to an apparatus for hydrolysis of a lignocellulosic material, wherein a mixture of the lignocellulosic material and an acidic water solution has a dry solid content of more than thirty percent and wherein at least parts of the apparatus are made from a material that comprises iron in an amount of at least fifty percent by weight and nickel in an amount of at least one point five percent by weight. The invention relates also to a method for extraction of saccharides from lignocellulosic materials by hydrolysis of the lignocellulosic materials in such an apparatus.

### BACKGROUND

Saccharides can be extracted from lignocellulose-containing materials, such as wood chips, sawdust and other fibrous materials from wood or plant. The saccharides, e.g. pentoses or hexoses, can then be converted into ethanol by fermentation. The process for extracting saccharides from a lignocellulosic material includes a hydrolysis reaction, in which the lignocellulosic material is mixed with an acidic water solution having a low pH value, e.g. in the interval of 0-4. The acidic water solution can, for example, be one with water highly diluted mineral acid, such as a diluted sulfuric acid. The hydrolysis is typically performed at high temperature, e.g. about or above 200 °C, and a challenge when performing the hydrolysis is that the combination of high temperature and low pH value makes the acidic water solution highly corrosive, which implies that apparatuses, such as reactor vessels, used in the hydrolysis process typically should be made from high-quality, corrosion-resisting materials, such as Ni-based alloys, tantalum, etc.

US 2012/0156748 A1 discloses the treatment of lignocellulosic material with chloride ions in order to reduce corrosion.

As is recognized in the European Patent No. 2132351 to van der Meulen et al., the presumed need for high-quality materials (and the considerable costs associated therewith) in the hydrolysis apparatuses used can hamper or even obstruct the industrial exploitation of the technologies and methods for extraction of saccharides from lignocellulosic materials. This European patent discloses, however, that relatively low-quality and thereby relatively inexpensive stainless steel materials actually can be used in the apparatuses employed for the hydrolysis process. The suggested reason for this unexpected finding is that the lignocellulose-containing raw materials release one or more chemical substances that work as protective agents (protectors or inhibitors) for the stainless steel materials used in the hydrolysis equipment. However, although this new finding represents a significant progress within the field, there are still problems to be solved. For example, since the industry typically wants to use different types of lignocellulosic materials (or the same lignocellulosic material but with varying characteristics, e.g. seasonal or geographical variations) as raw materials for the hydrolysis process, it is unsatisfactory that the preconditions for a problemfree use of the lignocellulosic raw materials together with the cheaper materials now suggested in the process apparatuses are not fully understood, not least since there are still considerable investments associated with, for instance, the start-up of a new process line for the extraction of saccharides from lignocellulosic materials.

An object of the present invention is therefore to present an improved and more reliable method for the extraction of saccharides from a lignocellulosic material by hydrolysis of the lignocellulosic material in an acidic water solution, which method clearly defines the preconditions for successful use of steel materials in the apparatuses used for performing the hydrolysis reaction, i.e. the preconditions under which the steel materials in the apparatuses do not corrode, or do not corrode more or faster than an acceptable corrosion rate. Another object is, by applying the method according to the present invention, expand the set of steel materials that can be used in apparatuses for performing the hydrolysis reaction to preferably include steel materials having an even lower quality and thereby being less expensive.

### SUMMARY OF THE INVENTION

The above-mentioned objects are achieved with a method and an apparatus according to the independent claims. Preferred embodiments are set forth in the dependent claims.

The invention relates to a method for extraction of saccharides from a lignocellulose-containing material, and comprises the following steps: mixing the lignocellulose-containing material with an acidic water solution to create a slurry having a pH value within the interval of 0-4; controlling the water content of the slurry to obtain a specific dry solid content of more than 30 %; and performing acid hydrolysis of the slurry in an apparatus, such as a reactor vessel, wherein at least parts of the apparatus that come into contact with the slurry are made from (of) a material that comprises iron in an amount of at least 50 % by weight and nickel in an amount of more than 1.5 % by weight.

As will be demonstrated below, an essential feature of the present invention is that the slurry entering the hydrolysis apparatus has a dry solid content of more than 30 %, and it is therefore necessary that the step of controlling the water content of the slurry entails adjusting the water content of the slurry. The step of controlling the water content of the slurry can, for example, comprise the step of dewatering the slurry to obtain such a relatively high dry solid content, wherein dewatering can be performed in, for example, a plug screw feeder. It should, however, be understood that if the slurry, i.e. the mixture of at least one lignocellulosic material and an acidic water solution, already has a dry solid content of more than 30 % after the mixing step, the step of controlling the water content of the slurry can merely imply ensuring that the slurry has a dry solid content of more than 30 %. In such a case, the step of controlling the water content of the slurry comprises the step of measuring the water content of the slurry.

The invention relates also to an apparatus, e.g. a reactor, for extraction of saccharides from a lignocellulose-containing material by acid hydrolysis of the lignocellulose-containing material, wherein the apparatus is configured for acid hydrolysis of a slurry, which is a mixture of the lignocellulose-containing material and an acidic water solution, having a pH value in the interval of 0-4 and a dry solid content of more than 30 %, and wherein at least parts of the apparatus that come into contact with the slurry are made from a material comprising iron in an amount of at least 50 % by weight and nickel in an amount of more than 1.5 % by weight. The apparatus of the present invention also comprises a detector for determining the water content of the slurry and a controller for controlling the water content of the slurry in response to a determined water content, the controller being arranged to control the water content of the slurry before acid hydrolysis takes place.

In preferred embodiments of the invention, the nickel content can be in the interval of 1.5 % to 10 % by weight, while the dry solid content of the slurry is in the interval of 30 % to 50 %, or even in the interval of 30 % to 55 %, although even higher dry solid contents are possible and within the scope of the invention. The acidic water solution can be a mineral acid, such as a sulphuric acid, a hydrochloric acid, or a nitric acid, or a mixture thereof, diluted with water to a pH value in the interval of 0-4, such that also the slurry, which is a mixture of the acidic water solution and the lignocellulosic material, has a pH value in the interval of 0-4. The acidic water solution can, however, also be an organic acid, such as a weak organic acid, e.g. a formic acid or an acetic acid, which, if necessary, is diluted to a pH value in the interval of 0-4.

As used herein, the terms "lignocellulose-containing material(s)" and "lignocellulosic material(s)" are used interchangeable and should be construed as having the same meaning. Further, apparatuses for extraction of saccharides from a lignocellulose-containing material comprise vessels, e.g. reactors, for performing the hydrolysis, but comprise also piping and feeding devices for transporting a slurry being a mixture of an acidic water solution and a lignocellulosic material. It should also be understood that it is not necessary that the whole apparatus is made from the specified material. Instead it is enough that the parts of the apparatus that come into contact with the slurry are made from a material comprising iron in an amount of at least 50 % by weight and nickel in an amount of more than 1.5 % by weight, which means, for example, that these parts are coatings, layers or linings on or at the relevant surfaces of an apparatus made from another type of material.

### DETAILED DESCRIPTION

In the aforementioned European Patent No. 2132351, a number of experimental results are presented. In these experiments, ten different materials were tested, where five materials were different grades of stainless steels and five were nickel-based alloys. Samples of the materials were cut to specific dimensions and were carefully weighed. The samples were then placed in an autoclave, and a sulphuric acid solution considerably diluted with water in the form of a saccharide-containing hydrolysate was added. The saccharide-containing hydrolysate, which was collected from a pilot plant for extraction of saccharides from wood chips, had a pH value of 1.67. The temperature in the autoclave was set to 210 °C and the pressure to 35 bar, and the hydrolysate was circulated in the autoclave for 13 days, whereupon the samples were weighed; and from the loss of weight, the corrosion rates (expressed in millimeters per year) were calculated.

From these results, and also from further experiments on a sub-selection of the samples, it was concluded that the rate of corrosion was acceptable for all materials tested except one; and that the rate of corrosion was, in particular, surprisingly low also for the most inexpensive steel grade (EN 1.4301) in the study. The experiments conducted and presented were not designed to find an explanation for these surprisingly low rates of corrosion also for the "simplest" and thereby cheapest materials, but the speculation and theory brought forward in EP2132351 is that since the acidic water solution, which in this case was a sulphuric acid solution, not only consists of sulphuric acid and water but also of smaller and greater amounts of different chemical substances that have been released from the lignocellulose-containing material, it is very likely that one or more of said released substances work as protective agent (protector or inhibitor) for the stainless steel materials tested.

From an industrial applicability point of view such an explanation is highly unsatisfactory, because, for instance, it is often desired to use different types of lignocellulosic materials as raw materials in an industrial plant for extraction of saccharides, and since different types of lignocellulosic materials are likely to release different types and amounts of substances that may or may not work as protective agents for the stainless steel materials used in the hydrolysis equipment, it creates an uncertainty whether or not a specific steel grade in practice will be corrosive resistant during acid hydrolysis of a specific type of lignocellulosic material or a mixture of several types of lignocellulosic materials. Needless to say, although EP2132351 discloses that relatively cheap steel materials can be used, the design and set up of a production line is still associated with considerable costs, and an inherent uncertainty regarding the corrosion rates and thereby the useful lifetimes for the apparatuses used in such a production line is often, or even usually, not acceptable.

The present invention therefore presents an improved and more reliable method for the extraction of saccharides from a lignocellulosic material (or a mixture of several types of lignocellulosic materials) by performing hydrolysis of the lignocellulosic material in an apparatus by mixing or contacting the lignocellulosic material with an acidic water solution to create a slurry having a pH value in the interval of 0-4, which method specifically takes into account not only the characteristics of the material (or materials) in the apparatus (or least in parts or in surfaces thereof) but also the characteristics of the lignocellulosic material, i.e. the characteristics that in practice create the preconditions for the corrosion resistance of the material(s) of which at least components or parts of said apparatus are made. The invention also relates to an apparatus for extraction of saccharides from a lignocellulosic material by performing hydrolysis of the lignocellulosic material in this apparatus, wherein both the properties of the material(s) in said apparatus and the properties of the lignocellulosic material(s) are specified, to make the apparatus or at least parts thereof corrosion resistant during said hydrolysis, or to provide the apparatus with a predetermined and acceptable rate of corrosion during said hydrolysis.

The apparatus for extraction of saccharides from a lignocellulosic material also comprises a a detector for determining the water content of the slurry and a controller for controlling the water content of the slurry in response to a determined water content, the controller being arranged to control the water content of the slurry before acid hydrolysis takes place. Thus, the detector and controller are arranged upstream from a vessel where acid hydrolysis takes place so that a slurry entering the apparatus passes the detector first and a water content is determined before the slurry passes to the controller. The controller suitably comprises a dewatering unit that decreases the water content of the slurry and a dilution unit where dilution fluid is supplied and a thorough mixing of the slurry and dilution fluid is performed. Depending on the determination of the water content of the slurry, the slurry is subjected to dewatering or dilution by the controller to achieve a desired water content.

After the water content of the slurry has been controlled in this way, the slurry passes into the vessel where acid hydrolysis takes place.

Below results from two experiments are presented, the purposes of which were to find and isolate the characteristics of a lignocellulosic material that effectively make an apparatus for performing hydrolysis of the lignocellulosic material in an acidic water solution corrosion resistant during said hydrolysis and, at the same time, to find the characteristics of the steel materials that are necessary for successful use in such an apparatus, and to expand the set of materials that can be used for successful use in such an apparatus.

### Example 1

Three different slurries, being mixtures of sawdust and sulphuric acid, were produced with 15 % dry substance (ds), 30 % ds, and 50 % ds. The slurry sample (of a specific percentage of dry substance) was placed in a steel reactor vessel, which in turn was heated by a copper vessel, which stood insulated in an autoclave furnace, and corrosion probes were placed in the slurry sample. The corrosion probes, also referred to as electrodes, were made from five different alloys: 316L (EN 1.4404), SAF 2205 (EN 1.4462), SAF 2507 (EN 1.4401), 904L (EN 1.4539) and the Ni-based super-alloy 625 (EN 2.4856), the chemical compositions of which can be found in Table 1 below.

The corrosion measurements, which were performed at 200 °C, utilized so-called LPR (Linear Polarisation Resistance) monitoring, which involves measurement of the polarisation resistance of a corroding electrode using a small amplitude (~25 mV) sinusoidal polarisation of the electrode. The results, which are taken from graphs obtained during the study, are summarized in Table 2 below.

**Table 1: Chemical compositions [in weight-%] for the alloys used in Example 1 and Example 2.**

| Alloy | Fe | Cr | Ni | Mo | Mn | Si | P | S | N | C |
|---|---|---|---|---|---|---|---|---|---|---|
| 316L | Bal | 17 | 12 | 2.5 | 2 | 0.75 | 0.045 | 0.03 | 0.1 | 0.03 |
| SAF 2205 | Bal | 22 | 5 | 3.2 | < 2 | < 1 | <0.03 | <0.015 | 0.018 | 0.03 |
| SAF 2507 | Bal | 25 | 7 | 4 | 1.2 | 0.8 | 0.035 | 0.015 | 0.3 | 0.03 |
| 904 L | Bal | 21 | 25.5 | 4.5 | 2 | 1 | 0.45 | 0.035 | Cu: 1.5 | |
| Alloy 625 | < 5 | 21.5 | Bal | 9 | 0.5 | 0.5 | 0.015 | 0.015 | Nb:0.1 | Al:0.4 |
| | | | | | | | | | Ta:4 | Co: 1 |
| LDX 2101 | Bal | 21.5 | 1.5 | 0.3 | 5 | | | | 0.22 | 0.03 |

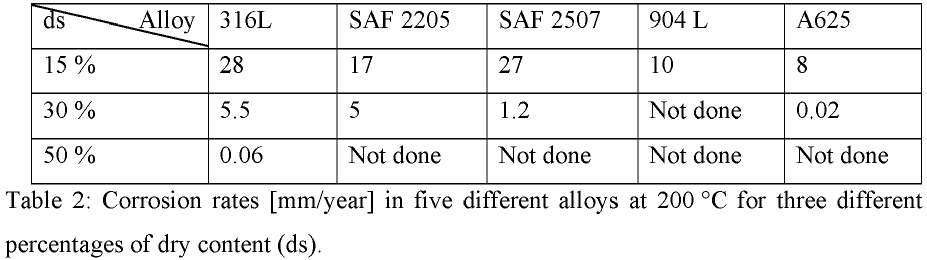

### Example 2

To verify the measurements done in Example 1, and to also investigate whether an even simpler steel grade can be used, the experiment above was repeated with LDX 2101 (EN 1.4162, see Table 1) and with SAF 2205 as reference sample. Three slurries, being mixtures of sawdust and diluted sulphuric acid, were prepared with three different dry solid contents: 15 % ds, 30 % ds and 50 % ds. The temperature was ramped up to 200 °C, where it was maintained. The test results are presented in Table 3 below.

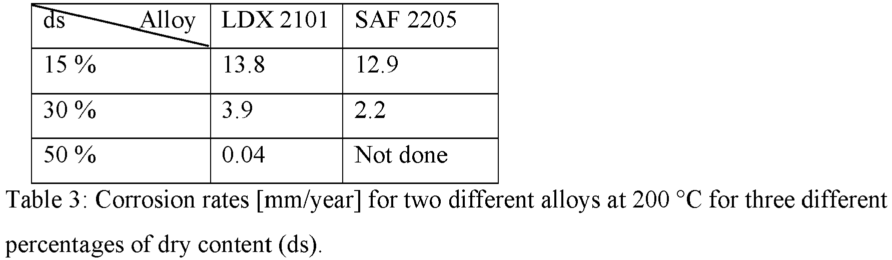

From Table 2 and Table 3 it is evident that the corrosion rates exhibit a clear dependence on the dry solid contents of the slurries, although a higher-quality steel grade generally corrodes less, as is expected. It can further be concluded that there is no linear dependence between the corrosion rate and the dry solid content of the slurry prepared, but instead it can be seen that for the highest dry solid content (i.e. a dry solid content of 50 %) there is essentially no corrosion taking place even in the simplest steel grades, e.g. in LDX 2101 and 316L. (Generally, a steel grade having a corrosion rate below 0.1 mm/year is classified as corrosion resistant.) Further, also for a dry solid content of 30 % in the slurry, the corrosion rates amount to a few millimeters per year also for the simplest steel grades, LDX 2101 and 316L, which - although presumably being too high for some applications - makes such relatively inexpensive steel grades interesting and useful as materials in apparatuses in, for example, pilot and test plants, which nevertheless are expected to have a relatively short useful life time, especially if the dry solid contents of the slurries introduced into these apparatuses actually are (at least somewhat) higher than 30 %. However, cheap metals having corrosion rates in the order of a few millimeters per year can be useful also in full scale plants, especially if regular maintenance and replacement of spent parts can be accepted. It can further be noted that all of the tested steel grades have an iron content of more than fifty percent. The simplest steel grade in the tests was LDX 2101, which is characterized by a nickel content of about 1.5 percent by weight, which therefore is considered to be the lowest useful nickel content for a material in an apparatus for acid hydrolysis of a slurry comprising a lignocellulosic material and an acidic water solution and having a pH value in the interval of 0-4 and a dry content of at least 30 %.

The present invention is based on the novel and surprising finding that the corrosion rate of a specific steel material, when used in an apparatus for acid hydrolysis of a lignocellulosic material (or a mixture of different types of lignocellulosic materials), is dependent on the water content of a slurry, being a mixture of the lignocellulosic material and an acidic water solution, introduced into the apparatus, wherein an acceptable corrosion rate during the acid hydrolysis is obtained when the slurry has a pH value in the interval of 0-4 and a specific dry solid content of more than 30 %, and the specific steel material comprises iron in an amount of at least 50 % by weight and nickel in an amount of at least 1.5 % by weight. In preferred embodiments of the invention, the nickel content can be in the interval of 1.5 % to 10 % by weight, while the dry solid content of the slurry is in the interval of over 30 % to 50 %, or even over 30 % to 55 %, although even higher dry solid contents are possible and within the scope of the invention. The acidic water solution can be a mineral acid, such as a sulphuric acid, a hydrochloric acid, a nitric acid, or a mixture thereof, diluted with water to a pH value in the interval of 0-4, such that also the slurry, which is a mixture of the acidic water solution and the lignocellulosic material, has a pH value in the interval of 0-4. The acidic water solution can, however, also be an organic acid, such as a weak organic acid, e.g. a formic acid or an acetic acid, which, if necessary, is diluted to a pH value in the interval of 0-4.

Finally, without being bound by theory, it is here submitted that when the lignocellulosic material that is used as raw material for the hydrolysis exhibits a high dry solid content, such that a slurry being a mixture of the lignocellulosic material in question and an acidic water solution also has a high dry solid content (i.e. over 30 %) then essentially all of the acidic water solution is absorbed by the lignocellulosic material, such that there is no (or very little) free water and no free (or relatively few) hydrogen ions that can create the excessive corrosion which previously was expected in this field.

Although the present invention has been described with reference to specific embodiments, it will be apparent to those skilled in the art that many variations and modifications can be done within the scope of the invention as described in the specification and defined with reference to the claims below.

## Claims

1. A method for extraction of saccharides from a lignocellulose-containing material, comprising the steps of:
mixing the lignocellulose-containing material with an acidic water solution to create a slurry having a pH value in the interval of 0-4;
controlling the water content of the slurry to obtain a specific dry solid content of the slurry; and
introducing the slurry into an apparatus and performing an acid hydrolysis of the slurry in the apparatus,
wherein the specific dry solid content of the slurry is more than 30 % and wherein at least parts of the apparatus that come into contact with the slurry are made of a material that comprises iron in an amount of at least 50 % by weight and nickel in an amount of at least 1.5 % by weight, and **characterized by** controlling the water content of the slurry comprising determining, using a detector, the water content of the slurry and controlling, using a controller, the water content of the slurry in response to a determined water content.

2. The method according to claim 1, wherein the step of controlling the water content of the slurry comprises dewatering of the slurry.

3. The method according to claim 1, wherein the step of controlling the water content of the slurry comprises measuring the dry solid content of the slurry.

4. An acid hydrolysis apparatus for extraction of saccharides from a lignocellulose-containing material, said apparatus being configured for acid hydrolysis of a slurry, which is a mixture of the lignocellulosic material and an acidic water solution and which has a pH value in the interval of 0-4 and a dry solid content of at least 30 %, wherein at least parts of the apparatus that come into contact with the slurry comprise iron in an amount of at least 50 % by weight and nickel in an amount of at least 1.5 % by weight, and **characterized by** comprising a detector for determining the water content of the slurry and a controller for controlling the water content of the slurry in response to a determined water content, the controller being arranged to control the water content of the slurry before acid hydrolysis takes place.

5. An acid hydrolysis apparatus according to claim 4, wherein the controller comprises a dewatering unit and a dilution unit.

## Patentansprüche

1. Verfahren für eine Extraktion von Sacchariden aus einem lignozellulosehaltigen Material, umfassend die Schritte:
Mischen des lignozellulosehaltigen Materials mit einer sauren Wasserlösung, um eine Aufschlämmung zu erzeugen, die einen pH-Wert in dem Intervall von 0-4 aufweist;
Steuern des Wassergehalts der Aufschlämmung, um einen spezifischen Trockenfeststoffgehalt der Aufschlämmung zu erhalten; und
Einführen der Aufschlämmung in eine Vorrichtung und Durchführen einer Säurehydrolyse der Aufschlämmung in der Vorrichtung,
wobei der spezifische Trockenfeststoffgehalt der Aufschlämmung mehr als 30 % beträgt und wobei mindestens Teile der Vorrichtung, die mit der Aufschlämmung in Berührung kommen, aus einem Material hergestellt sind, das Eisen in einer Menge zu mindestens 50 Gew.-% und Nickel in einer Menge zu mindestens 1,5 Gew.-% umfasst, und **gekennzeichnet durch** das Steuern des Wassergehalts der Aufschlämmung, umfassend ein Bestimmen, unter Verwendung eines Detektors, des Wassergehalts der Aufschlämmung und Steuern, unter Verwendung einer Steuerung, des Wassergehalts der Aufschlämmung als Reaktion auf einen bestimmten Wassergehalt.

2. Verfahren nach Anspruch 1, wobei der Schritt des Steuerns des Wassergehalts der Aufschlämmung ein Entwässern der Aufschlämmung umfasst.

3. Verfahren nach Anspruch 1, wobei der Schritt des Steuerns des Wassergehalts der Aufschlämmung ein Messen des Trockenfeststoffgehalts der Aufschlämmung umfasst.

4. Säurehydrolysevorrichtung für eine Extraktion von Sacchariden aus einem lignozellulosehaltigen Material, wobei die Vorrichtung für eine Säurehydrolyse einer Aufschlämmung konfiguriert ist, die eine Mischung aus dem Lignozellulosematerial und einer sauren Wasserlösung ist und die einen pH-Wert in dem Intervall von 0-4 und einen Trockenfeststoffgehalt von mindestens 30 % aufweist, wobei mindestens Teile der Vorrichtung, die mit der Aufschlämmung in Berührung kommen, Eisen in einer Menge zu mindestens 50 Gew.-% und Nickel in einer Menge zu mindestens 1,5 Gew.-% umfassen, und **dadurch gekennzeichnet, dass** sie einen Detektor zum Bestimmen des Wassergehalts der Aufschlämmung und eine Steuerung zum Steuern des Wassergehalts der Aufschlämmung als Reaktion auf einen bestimmten Wassergehalt umfasst, wobei die Steuerung angeordnet ist, um den Wassergehalt der Aufschlämmung zu steuern, bevor die Säurehydrolyse stattfindet.

5. Säurehydrolysevorrichtung nach Anspruch 4, wobei die Steuerung eine Entwässerungseinheit und eine Verdünnungseinheit umfasst.

## Revendications

1. Procédé d'extraction de saccharides à partir d'un matériau contenant de la lignocellulose, comprenant les étapes consistant à :
mélanger le matériau contenant de la lignocellulose avec une solution aqueuse acide pour créer une bouillie ayant une valeur de pH dans l'intervalle allant de 0 à 4 ;
réguler la teneur en eau de la bouillie pour obtenir une teneur en matières solides sèches spécifique de la bouillie ; et
introduire la suspension dans un appareil et réaliser une hydrolyse acide de la suspension dans l'appareil,
dans lequel la teneur en matières solides sèches spécifique de la bouillie est supérieure à 30 % et dans lequel au moins des parties de l'appareil qui entrent en contact avec la bouillie sont constituées d'un matériau qui comprend du fer en une quantité d'au moins 50 % en poids et du nickel en une quantité d'au moins 1,5 % en poids, et **caractérisé par** la régulation de la teneur en eau de la bouillie comprenant la détermination, à l'aide d'un détecteur, de la teneur en eau de la bouillie et la régulation, à l'aide d'un dispositif de contrôle, de la teneur en eau de la bouillie en réponse à une teneur en eau déterminée.

2. Procédé selon la revendication 1, dans lequel l'étape de régulation de la teneur en eau de la bouillie comprend la déshydratation de la bouillie.

3. Procédé selon la revendication 1, dans lequel l'étape de régulation de la teneur en eau de la bouillie comprend la mesure de la teneur en matières solides sèches de la bouillie.

4. Appareil d'hydrolyse acide pour l'extraction de saccharides à partir d'un matériau contenant de la lignocellulose, ledit appareil étant configuré pour l'hydrolyse acide d'une bouillie, qui est un mélange de la matière lignocellulosique et d'une solution aqueuse acide et qui a une valeur de pH dans l'intervalle allant de 0 à 4 et une teneur en matières solides sèches d'au moins 30 %, dans lequel au moins des parties de l'appareil qui entrent en contact avec la suspension comprennent du fer en une quantité d'au moins 50 % en poids et du nickel en une quantité d'au moins 1,5 % en poids, et **caractérisé par le fait qu'**il comprend un détecteur pour déterminer la teneur en eau de la bouillie et un régulateur pour réguler la teneur en eau de la bouillie en réponse à une teneur en eau déterminée, le dispositif de commande étant agencé pour réguler la teneur en eau de la bouillie avant que l'hydrolyse acide n'ait lieu.

5. Appareil d'hydrolyse acide selon la revendication 4, dans lequel le régulateur comprend une unité de déshydratation et une unité de dilution.
